# EUROPÄISCHE PATENTSCHRIFT

(11) **EP 1 508 047 B1**
(45) Veröffentlichungstag und Bekanntmachung des Hinweises auf die Patenterteilung: **06.08.2008**
(21) Anmeldenummer: 03735383.6
(22) Anmeldetag: 14.05.2003
(51) Int. Cl.: G01N 33/68, G01N 33/574, C07K 7/06, C12N 15/00, C12N 5/10, A61K 38/00, A61K 39/00

(54) **VERFAHREN ZUR IDENTIFIZIERUNG VON IMMUNREAKTIVEN PEPTIDEN**
METHOD FOR IDENTIFYING IMMUNOREACTIVE PEPTIDES
PROCEDE POUR IDENTIFIER DES PEPTIDES IMMUNOREACTIFS

(30) Priorität: 29.05.2002 DE 10225139
(43) Veröffentlichungstag der Anmeldung: 23.02.2005
(73) Patentinhaber: Immatics Biotechnologies GmbH, 72076 Tübingen (DE)
(72) Erfinder: WEINSCHENK, Toni, 73730 Esslingen (DE); RAMMENSEE, Hans, Georg, 72070 Tübingen-Unterjessingen (DE)
(74) Vertreter: Krauss, Jan
(86) Internationale Anmeldenummer: PCT/EP2003/005038
(87) Internationale Veröffentlichungsnummer: WO 2003/100432

(56) Entgegenhaltungen:
- WO-A-99/19349
- WO-A-03/102023
- WEINSCHENK TONI ET AL: "Integrated functional genomics approach for the design of patient-individual antitumor vaccines" CANCER RESEARCH, Bd. 62, Nr. 20, 15. Oktober 2002 (2002-10-15), Seiten 5818-5827, XP002266492 ISSN: 0008-5472
- SCHULTZE JOACHIM L ET AL: "From cancer genomics to cancer immunotherapy: Toward second-generation tumor antigens" TRENDS IN IMMUNOLOGY, Bd. 22, Nr. 9, September 2001 (2001-09), Seiten 516-523, XP004301120 ISSN: 1471-4906
- SCHIRLE MARKUS ET AL: "Identification of tumor-associated MHC class I ligands by a novel T cell-independent approach" EUROPEAN JOURNAL OF IMMUNOLOGY, WEINHEIM, DE, Bd. 30, Nr. 8, August 2000 (2000-08), Seiten 2216-2225, XP002246625 ISSN: 0014-2980 in der Anmeldung erwähnt
- MATHIASSEN SUSANNE ET AL: "Tumor-associated antigens identified by mRNA expression profiling induce protective anti-tumor immunity" EUROPEAN JOURNAL OF IMMUNOLOGY, Bd. 31, Nr. 4, April 2001 (2001-04), Seiten 1239-1246, XP002266493 ISSN: 0014-2980 in der Anmeldung erwähnt
- RAE FIONA K ET AL: "Novel association of a diverse range of genes with renal cell carcinoma as identified by differential display" INTERNATIONAL JOURNAL OF CANCER, Bd. 88, Nr. 5, 1. Dezember 2000 (2000-12-01), Seiten 726-732, XP002274523 ISSN: 0020-7136
- RENKVIST NICOLINA ET AL: "A listing of human tumor antigens recognized by T cells" CANCER IMMUNOLOGY IMMUNOTHERAPY, Bd. 50, Nr. 1, März 2001 (2001-03), Seiten 3-15, XP002274524 ISSN: 0340-7004

## Beschreibung

Die Offenbarung betrifft ein Verfahren zur Identifizierung und ein Verfahren zur Herstellung von immunreaktiven Peptiden und die so identifizierten/hergestellten immunreaktiven Peptide.

Derartige Peptide werden bspw. in der Immuntherapie von Tumorerkrankungen eingesetzt. Bei der Eliminierung von Tumorzellen durch das Immunsystem spielt die Erkennung von Tumor-assoziierten Antigenen (TAA) durch Komponenten des Immunsystems eine herausragende Rolle. Diesem Mechanismus liegt die Voraussetzung zugrunde, daß zwischen Tumorzellen und normalen Zellen qualitative oder quantitative Unterschiede bestehen. Um eine Anti-Tumor-Antwort herbeizuführen, müssen die Tumorzellen Antigene exprimieren, auf welche eine immunologische Antwort erfolgt, die für die Eliminierung des Tumors ausreicht.

Beteiligt bei der Abstoßung von Tumoren sind insbesondere CD8-exprimierende cytotoxische T-Lymphocyten (im folgenden CTL). Zur Auslösung einer derartigen Immunreaktion durch cytotoxische T-Zellen müssen den T-Zellen dazu fremde Proteine/Peptide präsentiert werden. T-Zellen erkennen Antigene als Peptidfragmente nur dann, wenn diese an Zelloberflächen von MHC-Molekülen präsentiert werden. Diese MHC-Moleküle ("major histocompatibility complex") sind Peptidrezeptoren, die normalerweise Peptide innerhalb der Zelle binden, um sie zu der Zelloberfläche zu transportieren. Dieser Komplex aus Peptid und MHC-Molekül kann durch die T-Zellen erkannt werden. Die MHC-Moleküle des Menschen werden auch als humane Leukocyten-Antigene (HLA) bezeichnet.

Die Behandlung von Krebs durch eine Immuntherapie, die Antigen-spezifisch ist und auf T-Zellen basiert, hat sich in der Vergangenheit als erfolgreich erwiesen.

Die Induktion einer spezifischen CTL-Antwort, die gegen einen Tumor gerichtet ist, ist von der Identifizierung von MHC-Klasse 1-Liganden abhängig, die von Tumor-assoziierten Antigenen abstammen (TAA). Solche Tumor-assoziierten Antigene können ausschließlich in bösartigen Zellen vorliegen, wie beispielsweise als Produkte von mutierten Genen. Andere wichtige Klassen von Tumor-assoziierten Antigenen sind Gewebe-spezifische Strukturen wie die CT ("cancer-testis") Antigene, eine dritte Klasse an Tumor-assoziierten Antigenen stellen Proteine dar, die in Tumoren überexprimiert werden.

Die Methoden zur Identifizierung und Charakterisierung von TAAs, die den Ausgangspunkt einer Tumorvakzine darstellen, basiert zum einen auf dem Einsatz von in Patienten bereits induzierten CTLs oder Antikörpern. Dieser immunologische Ansatz wird entweder mit dem Genexpressionsprofil oder mit einer massenspektrometrischen Sequenzierung der identifizierten Peptide kombiniert (siehe van der Bruggen et al., 1991, A gene encoding an antigen recognized by cytolytic T lymphocytes on a human melanoma, Science 254:1643-1647, und Cox et al., 1994, Identification of a peptide recognized by five melanoma-specific human cytotoxic T cell lines, Science 264:716-719). Methoden zur Identifizierung von TAAs, welche auf dem Vergleich des Transkriptionsprofils von Tumor- mit Normalgewebe basieren, sind bspw. Hybridisierung und der Einsatz von DNA-Chips.

Celis et al., 1994 , Induction of anti-tumor cytotoxic T lymphocytes in normal humans using primary cultures and synthetic peptide epitopes, Proc. Natl. Acad. Sci. USA 91:2105-2109, verwendeten ein Verfahren, das sich die Vorhersage von MHC-Klasse I-Liganden zunutze macht, die von einem ausgewählten Tumor-assoziierten Antigen abstammen, und bei dem anschließend diese Liganden als T-Zell-Epitope bestätigt wurden. Nachteilig an diesen Vorgehensweisen, die auf den T-Zellen von Patienten basieren, ist, dass die Kultivierung sehr aufwendig ist und dass man auf bereits existierende T-Zellen beschränkt ist.

Bekannt ist weiterhin ein Ansatz, der nicht von T-Zellen abhängig ist und bei dem die Epitop-Vorhersage mit den Screenen nach vorhergesagten Peptiden in komplexen Peptid-Mischungen kombiniert wird, wobei die Peptide durch hochsensitive kapillare Flüssigchromatographie/Massen-Spektroskopie (LC-MS) identifiziert wurden (siehe Schirle et al., 2000, Identification of tumor-associated MHC class I ligands by a novel T-cell independent approach, Eur. J. Immunol. 30:2216-2225).

Mit der DNA-Microarray-Technologie wird ein neuer Ansatz geboten, mit dem das Expressionsprofil eines Tumors und das des korrespondierenden autologen normalen Gewebes verglichen werden kann. So zeigten z.B. Young et al., 2001, Expression profiling of renal epithelial neoplasms: a method for tumor classification and discovery of diagnostic molecular markers, Am J. Pathol. 158:1639-1651, dass mit diesem Verfahren eine große Anzahl von Tumor-assoziierten Antigenen aus einzelnen Tumoren identifiziert werden kann. MHC-I-Liganden, die von überexprimierten oder selektiv exprimierten Proteinen abstammen, stellen somit potentielle Ziele für eine spezifische CTL-Erkennung von Tumoren dar. Mathiassen et al., 2001, Tumor-associated antigens identified by mRNA expression profiling induce protective anti-tumor immunity, Eur. J. Immuno. 31:1239-1246 konnten im Mausmodell zeigen, dass es mit einer kombinierten Expressionsanalyse/Epitopvorhersage möglich ist, eine wirksame Vakzine herzustellen.

Diese Epitop-Vorhersage weist jedoch den Nachteil auf, dass selbst für nur wenige Zielgene eine riesige Anzahl von möglichen Peptiden bestimmt werden, von denen die Mehrheit tatsächlich gar nicht durch MHC-Moleküle präsentiert wird, und die somit auch keine CTL-Antwort auslösen können.

Daher ist es Aufgabe der vorliegenden Erfindung, ein neues in vitro Verfahren bereitzustellen, mit dem auf einfache Weise und gezielt immunreaktive Peptide identifiziert werden können.

Diese Aufgabe wird erfindungsgemäß gelöst durch ein in vitro Verfahren zur Identifizierung von MHC-Liganden mit den Schritten:
(a) Bereitstellung einer Probe aus tumorösem und korrespondierendem gesunden Gewebe,
(b) Bestimmung des tumorspezifischen Expressionsprofils aus der bereitgestellten Probe,
(c) Isolation und Analyse von MHC-Liganden aus der Probe aus tumorösem Gewebe,
(d) Abgleich der aus Schritt (b) und (c) gewonnenen Daten, und
(e) Identifizierung von MHC-Liganden auf der Basis der abgeglichenen Daten.
   Die Erfinder haben erkannt, dass durch die Kombination einer Expressionsanalyse mit den isolierten und analysierten antigenen Peptiden eines Tumors spezifisch Kandidaten für eine individuelle Vakzine identifiziert werden können.

Durch die Isolierung der antigenen Peptide und den Abgleich mit dem Expressionsprofil von Genen eines tumorösen Gewebes wird vermieden, dass eine extrem große Menge an potentiell immunreaktiven Peptiden gewonnen wird. Vielmehr werden gezielt Peptide identifiziert, die tatsächlich von MHC-Molekülen präsentiert werden und daher als immunreaktive Peptide geeignet sind.

Mit dem erfindungsgemäßen Verfahren ist es daher möglich, jeweils Patienten-individuelle Peptide zu identifizieren, also die bspw. als Vakzine einzusetzenden Peptide genau auf den Patienten abzustimmen, um eine gezielte Immunantwort auszulösen.

Dieses Verfahren kann - nach Erhalt von Patientenproben - systematisch und effizient bspw. von Großlabors durchgeführt werden, die nach erfolgter Identifizierung geeigneter immunreaktiver Peptide deren Sequenzen an die behandelnden Kliniken weitergeben, wo die Peptide synthetisiert und appliziert werden. Es ist aber auch möglich, dass ein Labor sowohl die Identifizierung als auch die Herstellung der für den jeweiligen Patienten geeigneten Peptide vornimmt.

Das neue Verfahren ist also sowohl im Rahmen einer reinen Dienstleistung als auch kombiniert mit der Lieferung der identifizierten immunreaktiven Peptide einsetzbar.

Die in Schritt (c) isolierten Peptide sind MHC-Liganden.

Nur an MHC-Moleküle gebundene Peptide können eine zelluläre Immunantwort auslösen. Peptide, die bspw. von überexprimierten Genen in einem Tumor abstammen, aber nicht an MHC-Moleküle gebunden sind, lösen keine CTL-Immunreaktion aus. Daher sind nicht alle, z.B. lediglich durch Epitopvorhersage identifizierten Peptide immunreaktiv.

In einer weiteren bevorzugten Ausführungsform wird der Schritt (b) mittels einer Mikroarray-Analyse und/oder einer reversen Transkriptions-Polymerase-Kettenreaktion durchgeführt.

Bei der Mikroarray-Analyse wird unter Verwendung bestimmter DNA- oder Genchips das Expressionsprofil von Tumorgewebe mit korrespondierendem normalem Gewebe abgeglichen und selektiv exprimierte oder überexprimierte Gene identifiziert. Dieses Verfahren ist im Stand der Technik gut bekannt und ist beispielsweise beschrieben in Schena et al., 1995, Quantitative monitoring of gene expression patterns with a complementary DNA microarray, Science 270:467-470.

Die reverse Transkriptions-Polymerase-Kettenreaktion (im folgenden auch RT-PCR) kann dazu ausgenützt werden, um die Expression eines Gens zu quantifizieren.

Dazu wird aus RNA, die bspw. aus Tumorzellen isoliert wurde, cDNA gewonnen und diese anschließend als "Template" für eine PCR verwendet. Somit kann anhand der amplifizierten DNA verglichen werden, welche Gene wie stark transkribiert wurden.

In einer bevorzugten Ausführungsform wird in Schritt (c) die Analyse mittels Massenspektrometer durchgeführt.

Mittels dieser Technik können die einzelnen Peptide genau und effizient in einem großen Durchsatz identifiziert werden. Die Anwendung der Massenspektroskopie zur Identifizierung von Peptiden aus Tumorgewebe ist bspw. beschrieben in Schirle et al., 2000, Identification of tumor-associated MHC class I ligands by a novel T-cell independent approach, Eur. J. Immunol. 30:2216-2225.

In einer weiteren bevorzugten Ausführungsform werden im Schritt (c) unter Verwendung geeigneter Datenbanken aufgrund des Expressionsprofils potentielle antigene Peptide vorhersagt und das Massenspektrometer auf diese vorhergesagten Antigene kalibriert.

Die Verwendung von Datenbanken zur Vorhersage von potentiellen Antigenen und die Anwendung der hieraus gewonnenen Daten ist bspw. beschrieben in Schirle et al., 2001, Combining computer algorithms with experimental approaches permits rapid and accurate identification of T-cell epitopes from defined antigens, J. Immunol. Methods, 257:1-16. Beispiele für Datenbanken sind bspw. unter http://www.syfpeithi.de und http://www.paproc.de zu finden.

In einer weiteren bevorzugten Ausführungsform des Verfahrens wird nach Schritt (c) ein weiterer Schritt durchgeführt, bei dem die Reaktivität von peripheren Leukocyten, vorzugsweise von T-Leukocyten gegen die isolierten antigenen Peptide getestet wird.

In einer bevorzugten Ausführungsform wird die Reaktivität von peripheren Leukocyten gegen die isolierten antigenen Peptide über die Messung der von den Leukocyten synthetisierten γ-Interferon-mRNA und/oder Zytokin-mRNA getestet.

Durch den Nachweis von γ-Interferon- oder Zytokin-mRNA ist es möglich, die spezifische Reaktivität von Leukocyten, vorzugsweise von T-Lymphocyten gegenüber den antigenen Peptiden genau nachzuweisen. Die beiden Stoffe werden von aktivierten T-Lymphocyten nach ihrer Aktivierung durch korrespondierende antigene Peptide sekretiert.

Dieser zusätzliche Schritt bietet die Möglichkeit, noch gezielter Kandidaten aus den bereits identifizierten Peptiden zu identifizieren.

In einer anderen bevorzugten Ausführungsform des Verfahrens wird nach Schritt (c) ein weiterer Schritt durchgeführt, bei dem die Existenz spezifischer T-Lymphocyten nachgewiesen wird.

Mit diesem Verfahren ist es möglich, spezifisch herauszufinden, inwieweit und in welchem Umfang T-Lymphocyten bereits im Patienten gegen die isolierten und die identifizierten Peptide vorliegen. Durch diesen Schritt ist es möglich, auch nur diejenigen Peptide als Vakzine einzusetzen, für die bereits T-Lymphocyten im Patienten vorliegen. Die Peptide können dann dazu eingesetzt werden, diese spezifischen T-Lymphocyten zu aktivieren.

In einem weiteren bevorzugten Verfahren erfolgt der Nachweis der Existenz spezifischer T-Lymphocyten über die Markierung der Leukocyten mit rekonstituierten Komplexen aus Antigen-präsentierenden Molekülen und antigenem Peptid.

Bei diesem Verfahren wird die so genannte Tetramer-Technologie eingesetzt. Die Gewinnung solcher rekonstituierten Komplexe ("Tetramere") und deren Einsatz ist bspw. beschrieben in Altman et al., 1996, Phenotypic analysis of antigen-specific T-lympnocytes, Science 274:94-96.

Es werden weiterhin immunreaktive Peptide beschrieben, die durch das erfindungsgemäße Verfahren identifiziert und/oder hergestellt werden.

Diese Peptide können nach Identifizierung gezielt und spezifisch für den einzelnen Patienten hergestellt werden.

Es wird weiterhin eine pharmazeutische Zusammensetzung beschrieben, die eines oder mehrere der Peptide enthält, die nach dem erfindungsgemäßen Verfahren identifiziert und/oder hergestellt wurden.

Diese Zusammensetzung dient beispielsweise der parenteralen Verabreichung, beispielsweise durch subkutane, intradermale oder intramuskuläre Verabreichung, oder der oralen Verabreichung. Dabei sind die Peptide in einem pharmazeutisch annehmbaren, vorzugsweise wässrigen, Träger gelöst oder suspendiert, darüber hinaus kann die Zusammensetzung Hilfsstoffe, wie beispielsweise Puffer, Bindemittel, Verbindungsmittel, etc. enthalten. Die Peptide können auch zusammen mit immunstimulierenden Substanzen, wie z.B. Cytokinen verabreicht werden. Eine umfassende Darstellung von Hilfsstoffen, wie sie bei einer derartigen Zusammensetzung verwendet werden können, ist beispielsweise in A. Kibbe, Handbook of Pharmaceutical Excipients, 3. Ed., 2000, American Pharmaceutical Association and Pharmaceutical Press, dargestellt.

Das Peptid kann zur Behandlung von Tumorerkrankungen und zur Herstellung eines Mittels zur Behandlung von Tumorerkrankungen verwendet werden.

Die zu behandelnden Tumorerkrankungen umfassen dabei Nieren-, Brust-, Pankreas-, Magen-, Hoden- und/oder Hautkrebs. Die Aufzählung der Tumorerkrankungen ist dabei lediglich beispielhaft und soll den Verwendungsbereich nicht eingrenzen.

Ferner kann das Peptid auch zur Beurteilung eines Therapieverlaufs bei einer Tumorerkrankung eingesetzt werden.

Auch bei anderen Immunisierungen oder Therapien kann das Peptid für das Monitoring einer Therapie eingesetzt werden. Somit ist das Peptid nicht nur therapeutisch, sondern auch diagnostisch einsetzbar.

Weiterhin können die Peptide zur Herstellung eines Antikörpers eingesetzt werden.

Polyklonale Antikörper können in herkömmlicher Weise durch Immunisierung von Tieren mittels Injektion der Peptide und anschließender Reinigung des Immunglobulins gewonnen werden.

Monoklonale Antikörper können nach Standardprotokollen hergestellt werden, wie beispielsweise in Methods Enzymol. (1986), 121, Hybridoma technology and monoclonal antibodies, beschrieben.

Es werden weiterhin Nukleinsäuremoleküle, kodierend für das mit dem erfindungsgemäßen Verfahren isolierte Peptid, beschrieben.

Die Nukleinsäuremoleküle können dabei DNA- oder RNA-Moleküle sein und ggf. auch für die Immuntherapie von Krebserkrankungen eingesetzt werden.

Die Nukleinsäuremoleküle können auch in einem Vektor vorliegen.

Weiterhin wird eine Zelle beschrieben, die mit Hilfe des Nukleinsäuremoleküls genetisch so verändert wurde, dass sie ein identifiziertes Peptid produziert.

Weiterhin wird ein Verfahren zur Herstellung eines immunreaktiven Peptids beschrieben, bei dem nach dem beschriebenen Verfahren ein Peptid identifiziert und das identifizierte Peptid chemisch, in vitro oder in vivo synthetisiert wird.

Peptide können durch chemische Verknüpfung von Aminosäuren bspw. durch das im Fachgebiet bekannte Verfahren nach Merrifield hergestellt werden (siehe Merrifield RB, 1963, J. Am. Chem. Soc. 85:2149-2154).

In vitro lassen sich Peptide z.B. in zellfreien Systemen herstellen, in vivo unter Verwendung von Zellen.

Es wird weiterhin ein Verfahren zur Herstellung eines Impfstoffs beschrieben, mit den Schritten
a) Durchführung des oben beschriebenen Verfahrens,
b) Herstellung der identifizierten immunreaktiven Peptide und
c) Formulierung der hergestellten immunreaktiven Peptide.

Ausführungsbeispiele der Erfindung werden nachfolgend in den Figuren und dem Beispiel dargestellt und erläutert. Es zeigen:
- Fig. 1: die Expressions-Analyse ausgewählter Gene mittels quantitativer RT-PCR;
- Fig. 2: die Detektion von Keratin 18-spezifischen CD8⁺ T-Lymphocyten.

### Beispiel

### Patientenproben

Von der Abteilung für Urologie, Universität Tübingen, wurden zwei Proben erhalten, die von Patienten stammten, die histologisch bestätigte Nierenzelltumore aufwiesen. Beide Patienten hatten keine präoperative Therapie erhalten. Patient Nr. 1 (im folgenden mit RCC01 bezeichnet) besaß die folgende HLA-Typisierung: HLA-A*02 A*68 B*18 B*44; der Patient Nr. 2 (im folgenden mit RCC13 bezeichnet) HLA-A*02 A*24 B*07 B*40.

### Isolierung der MHC-Klasse-I-gebundenen Peptide

Die schockgefrorenen Tumorproben wurden prozessiert wie bereits beschrieben in Schirle, M. et al., Identification of tumor-associated MHC class I ligands by a novel T cell-independent approach, 2000, European Journal of Immunology, 30:2216-2225. Die Peptide wurden nach Standardprotokollen isoliert, und zwar unter Verwendung des monoklonalen Antikörpers W6/32, der spezifisch für die HLA-Klasse-I-Moleküle ist, oder des monoklonalen Antikörpers BB7.2, der für HLA-A2 spezifisch ist. Barnstable, C.J. et al., Production of monoclonal antibodies to group A erythrocytes, HLA and other human cell surface antigens-new tools for genetic analysis, 1978, Cell, 14:9-20 und Parham, P. & Brodsky, F.M., Partial purification and some properties of BB7.2. A cytotoxic monoclonal antibody with specificity for HLA-A2 and a variant of HLA-A28, 1981, Hum. Immunol., 3:277-299, beschrieben die Herstellung und Anwendung dieser Antikörper.

### Massenspektroskopie

Peptide, die aus dem Tumorgewebe des Patienten RCC01 gewonnen wurden, wurden durch "reversed phase HPLC" getrennt (SMART-System, µRPC C2/C18 SC 2.1/19, Amersham Pharmacia Biotech) und die erhaltenen Fraktionen durch nanoESI MS analysiert. Dabei wurde vorgegangen, wie beschrieben in Schirle, M. et al., Identification of tumor-associated MHC class I ligands by a novel T cell-independent approach, 2000, European Journal of Immunology, 30:2216-2225.

Die Peptide, die aus Tumorgewebe des Patienten RCC13 gewonnen wurden, wurden wie eben erwähnt durch Kapillar-LC-MS identifiziert, allerdings mit geringfügigen Änderungen: 100 µl der Proben wurden jeweils geladen, entsalzt und auf einer 300 µm * 5 mm C18 µ-Vorsäule (LC Packings) vorkonzentriert. Das Lösungsmittel und die Probe wurden mittels einer Spritzenpumpe (PHD 2000, Harvard Apparatur, Inc.) mit einer abgedichteten 100 µl-Spritze (1710 RNR, Hamilton) mit einer Geschwindigkeit von 2 µl/min zugeführt. Zur Trennung der Peptide wurde die Vorkonzentrierungssäule vor eine 75 µm * 250 mm C-18-Säule (LC Packings) geschaltet. Anschließend wurde ein binärer Gradient mit 25-60% B innerhalb von 70 min gefahren, wobei die Flussrate von 12 µl/min auf ungefähr 300 nl/min reduziert wurde, und zwar unter Verwendung eines TEE-Anschlusses (ZT1C, Valco) und einer 300 µm * 150 mm C-18-Säule.

Um sicherzustellen, dass das System frei von restlichen Peptiden war, wurde jeweils eine Leer-Probe gemessen. Online-Fragmentierung wurde wie beschrieben durchgeführt, und die Spektren der Fragmente wurden manuell analysiert.

Die Datenbankrecherchen (NCBInr, EST) wurden unter Verwendung von MASCOT durchgeführt (http://www.matrixscience.com).

### Gewinnung der RNA

Fragmente von normalem und bösartigem Nierengewebe wurden aufgeteilt, schockgefroren und unter Flüssigstickstoff mittels eines Mörsers und eines rotierenden Homogenisierers (Heidolph-Instrumente) in TRIZOL (Life Technologies) homogenisiert. Die Gesamt-RNA wurde nach dem Protokoll des Herstellers aufgereinigt und anschließend mit RNeasy (QIAGEN) weiter gereinigt. Gesamt-RNA von menschlichen Geweben wurde bei Clontech erworben (Human total RNA Master Panel II).

### High-Density Oligonukleotid-Microarray-Analyse

Unter Verwendung der Superscript RT II (Life Technologies) reversen Transkriptase wurde doppelsträngige DNA aus 40 µg der Gesamt-RNA synthetisiert. Die dazu verwendeten Primer (Eurogentec) waren durch das Protokoll von Affymetrix vorgegeben. *In vitro*-Transkription wurde unter Verwendung des BioArray^{™} High Yield^{™} RNA Transkript Labeling Kit (ENZO Diagnostics, Inc.) durchgeführt, anschließend erfolgte eine Fragmentierung und Hybridisierung auf Genchips von Affymetrix (HuGeneFL). Die Färbung erfolgte mit Streptavidin-Phycoerythrin und biotinyliertem Anti-Streptavidin-Antikörper, wobei nach dem Protokoll des Herstellers vorgegangen wurde (Affymetrix).

Die Daten wurden unter Verwendung eines Affymetrix GeneArray Scanners mit der Microarray Analysis Suite 4.0 Software analysiert.

### Real-Time RT-PCR

Die cDNA, die für die Microarrays hergestellt wurde, wurde auch für die quantitative PCR-Analyse eingesetzt.

Für jedes Gen wurde ein zweifacher Ansatz gefahren (40 Zyklen, 95°C x 15 s, 60°C x 1 min), wobei das ABI PRISM 7700 Sequenzen-Detektions-System (Applied Biosystems) unter der Verwendung von SYBRGreen eingesetzt wurde. Die Proben wurden unabhängig voneinander zwei- bis dreimal analysiert. Die Primer (MWG-Biotech) waren zu beiden Seiten eines Introns gewählt, die PCR-Effizienzen wurden für alle Primerpaare getestet und erwiesen sich jeweils als ungefähr 1.

Die PCR-Produkte wurden auf 3%-igen Agarosegelen auf Reinheit analysiert. Nach Klonierung in den pCR4-TOPO-Vektor unter Verwendung des TOPO TA Cloning Kit (Invitrogen) wurden ihre Sequenzen verifiziert. Die Datenanalyse umfasste das delta C_{T}-Verfahren der relativen Quantifizierung.

### Laser Capture Microdissection

Eingebettete schockgefrorene Gewebeproben wurden mit einer Dicke von 6 µm geschnitten und für ungefähr 15 min in 70%-iges Ethanol überführt. Die Objektträger wurden 90 s in Mayer's Hematoxylin (Merck) inkubiert, mit Wasser abgewaschen und 1 min in 70%-igem Alkohol inkubiert, anschließend 1 min in 95%-igem Alkohol, 30 s in 1% alkoholischem Eosin Y (Sigma), 2 x 2 min in 95%-igem Ethanol, 2 x 2 min in 100%-igem Ethanol und schließlich 2 x 5 min in Xylen. Nach einer 15-minütigen Lufttrocknung wurden die Objektträger unter trockenen Bedingungen aufbewahrt. Normale epitheliale tubuläre Zellen und Karzinomzellen wurden unter Verwendung des PixCell II LCM Systems (Arcturus Engineering) durch Laser Capture Microdissection (LCM) isoliert. Die Gesamt-RNA wurde in 400 µl TRIZOL extrahiert.

### PBMC, Tetramer-Produktion und Durchflußcytometrie

Von zwei gesunden Blutspendern (HD1 und HD2), die serologisch als CMV-positiv typisiert wurden, wurden periphere blutmononucleäre Zellen (im folgenden PBMC) durch Gradientenzentrifugation (FicoLite H) isoliert und eingefroren.

HLA-A*0201-Tetramerkomplexe wurden wie bei Altman et al., 1996, Phenotypic analysis of antigen-specific T-lympnocytes, Science 274:94-96, beschrieben, folgendermaßen hergestellt: Die HLA-A2 bindenden Peptide, die zur Rekonstituierung eingesetzt wurden, waren ALLNIKVKL von Keratin 18 und NLVPMVATV von pp65 HCMVA. Die Tetramere wurden durch Vermischung der biotinylierten Monomere mit Streptavidin-PE oder Streptavidin-APC hergestellt und 2-3 x 10⁶ Zellen wurden 30 min lang bei 4°C mit beiden Tetrameren inkubiert: 10 µg/ml für jedes Monomer in PBS, 0,01% NaN₃, 2 mM EDTA, 50% fetales Kälberserum. Anschließend wurden die monoklonale Antikörper Anti-CD4-FITC (Coulter-Immunotech) und Anti-CD8-PerCP (Becton Dickinson) für 20 min hinzugefügt. Nach drei Waschschritten wurden die Proben in FACS-Puffer und 1% Formaldehyd fixiert. Eine Vierfarbenanalyse wurde auf einem FACS-Caliburcytometer (Becton Dickinson) durchgeführt.

### Ergebnisse

Die Expression von ungefähr 7000 Genen in Tumoren und korrespondierendem normalem Gewebe zweier Nierenzelltumore wurde analysiert. Es konnte herausgefunden werden, dass im Tumorgewebe zwischen 400 und 500 Gene überexprimiert oder selektiv exprimiert waren. 70 Gene waren in den Tumoren beider Patienten überexprimiert. In dem Patienten 1 waren 268 überexprimierte und 129 exklusiv exprimierte Gene detektiert worden. Die meisten der überexprimierten Gene sind Krebs-assoziiert, d.h. also entweder Onkogene, Tumorsuppressorgene oder Gene, welche bereits bei Krebs als überexprimiert beschrieben wurden, wie beispielsweise CCND1, CA9, Cerebrosidsulfotransferase und das Parathyroidhormon-ähnliche Hormon. Das Krebs-assoziierte "adipose differentiationrelated" Protein (ADFP) oder Adipophilin zeigte den zweithöchsten Grad an Überexpression. Von diesem Protein konnte darüber hinaus auch gezeigt werden, dass es gegenüber normalem Gewebe anderer Organe, also nicht nur gegenüber normalem Nierengewebe, im Tumorgewebe stark überexprimiert war.

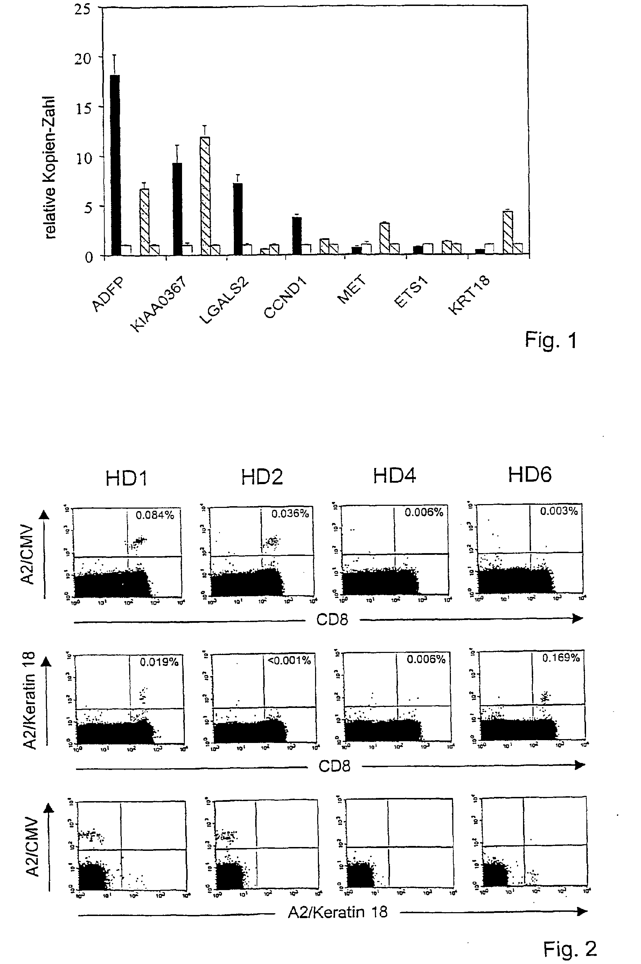

Um die durch die Mikroarray-Analyse erhaltenen Daten zu verifizieren, wurde die Expression von ausgewählten Genen durch quantitative PCR analysiert. In Fig. 1 ist die Expressions-Analyse ausgewählter Gene mittels RT-PCR dargestellt. Die RT-PCR wurde unter Verwendung der gleichen cDNA durchgeführt, die auch bei der Microarray-Analyse eingesetzt wurde. Die Kopienzahl ist relativ zur 18S rRNA angegeben und bezüglich normalem Gewebe (=1) jedes Patienten normalisiert. Die schwarz ausgefüllten Balken geben die Zahlen im Tumorgewebe des Patienten RCC01 wieder, die weiß ausgefüllten Balken die des Normalgewebes und die schräg gestreift und grau ausgefüllten Balken die Zahlen im Tumorgewebe des Patienten RCC13.

Es zeigte sich, dass die durch Microarray belegte Überexpression von Adipophilin (ADFP) und Cyclin D1 (CCND1) durch quantitative PCR bestätigt werden konnte. Ebenso konnte bestätigt werden, dass ets-1 (ETS1) in normalem und Tumor-Gewebe gleich stark exprimiert wird. Außerdem waren die relativen Expressionslevel, die durch beide Techniken detektiert wurden, ungefähr vergleichbar.

So war Adipophilin in der Microarray-Analyse im Tumorgewebe des Patienten RCC01 mit einem Faktor von 29,1 überexprimiert, in der quantitativen PCR um 18,1 (siehe Fig.1). Beim Patienten RCC13 wurde in der Microarray-Analyse ein Faktor von 11,4 und in der quantitativen PCR ein Faktor von 6,7 (siehe Fig.1) gefunden. Galectin 2 (LGALS2) war im Patienten RCC01 überexprimiert, Keratin-18 (KRT18) im Patienten RCCI3. Eine Ausnahme in der Übereinstimmung der Daten aus der Microarray-Analyse mit den Daten der quantitativen PCR bildete die Überexpression von KIAA0367 und met-Protoonkogen (MET) im Patienten RCC 13.

### Identifizierung der MHC Klasse I-Liganden

Aus den Tumorgeweben konnten insgesamt 85 Liganden gewonnen werden, die an die HLA-Subtypen HLA-A*02, HLA-A*68, HLA-B*18 oder HLA-B*44 gebunden waren. Peptide, die an HLA-A*02 banden, wiesen das allel-spezifische Peptidmotiv (Leucin, Valin, Isoleucin, Alanin, Methionin an Position 2, Leucin, Valin, Isoleucin oder Alanin am C-Terminus) auf. Die meisten Liganden stammten von so genannten "Houskeeping"-Proteinen, jedoch konnten auch Liganden von Proteinen mit erwiesener Tumor-Assoziierung detektiert werden, wie bspw. YVDPVITSI vom met-Protoonkogen, ALLNIKVKL von Keratin 18 und SVASTITGV von Adipophilin.

HLA-A*68 Liganden wurden durch deren Ankeraminosäuren Threonin, Isoleucin, Valin, Alanin oder Leucin an Position 2 und Arginin oder Lysin am C-Terminus identifiziert. Unter den HLA-A*68 Liganden konnten zwei weitere Peptide von Adipophilin identifiziert werden: MTSALPIIQK und MAGDIYSVFR. Die Liganden mit Glutaminsäure an Position 2 wurden HLA-B*44 zugeordnet, das Peptidmotiv von HLA-B*18 ist bisher noch unbekannt, weswegen eine Zuordnung der Liganden dieser beiden HLA-B-Moleküle nicht möglich war.

Der Abgleich der Mikroarray-Daten mit den isolierten Liganden zeigte, dass 10 überexprimierte Gene als Quellen für MHC-Liganden dienten: Adipophilin, KIAA0367, SEC14-like 1, B-Zelltranslokationsgen 1, Aldolase A, Cyclin D1, Annexin A4, Catenin alpha 1, Galectin 2 und LMP2. Drei dieser Gene sind bereits in der SEREX-Datenbank aufgeführt, dies sind KIAA0367, Aldolase A und Catenin alpha 1.

Ein besonders interessanter Ligand konnte bei dem Patienten RCC 13 identifiziert werden (ALAAVVTEV), der durch ein "Reading Frame" kodiert wird, der im Vergleich zum DEAD/H-Box Polypeptid 3 (DDX3) um ein Nukleotid verschoben war. ALAAVVTEV wird von den Nukleotiden 317-343 des kodierenden Strangs von DDX3 kodiert, wohingegen die Nukleotide 316 bis 342 für GIGSRGDRS des DDX3 Proteins kodieren.

### Nachweis von spezifischen T-Zellen in normalem CD8⁺ T-Zell-Repertoire

PBMC von 6 HLA-A2 positiven Nierenzell-Tumorpatienten wurden auf eine Reaktion gegen vier der relevanten Peptide getestet: Dies waren HLA-A*02-gebundene Liganden von Adipophilin, Keratin 18, KIAA0367 und met-Protoonkogen. Dazu wurde ein sehr sensitives quantitatives PCR-Assay verwendet, um nach einer 7-tätigen *in vitro-*Sensibilisierung mit dem Peptid die γ-Interferon-mRNA-Produktion durch die CD8⁺ T-Zellen nachzuweisen. Nach Stimulierung mit met-Protoonkogen-, Keratin 18- oder Adipophilin-Peptiden konnten sporadische Antworten verzeichnet werden.

Die PBMC-Färbung von an Tumor erkrankten und gesunden Individuen mit HLA-A*0201 Tetrameren erfolgte entweder mit Tetrameren, die mit Adipophilin konstituiert waren, mit Keratin 18 oder mit met-Protoonkogen.

In Fig. 2 ist die Detektion von Keratin 18-spezifischen T-Lymphozyten gezeigt. Dazu wurden PBMC von vier gesunden HLA-A*02⁺ Spendern (HD1, 2, 4, 6) gleichzeitig mit HLA-A2/Keratin 18-PE Tetrameren, HLA-A2/CMV-APC Tetrameren, CD8-PerCP und CD4-FITC gefärbt. Die Dot Plots zeigen die Ergebnisse von 1 bis 3 unabhängigen Experimenten für 1 x 10⁶ PBMC. In den Plots ist der Prozentsatz der Tetramer+-Zellen in der CD8⁺ CD4⁻ -Population dargestellt.

Unerwarteterweise konnte eine signifikante Population von CD8⁺ T-Lymphocyten spezifisch für Keratin 18 (zwischen 0,02% und 0,2% der CD8⁺ T-Zellen) in 4 von 22 gesunden Individuen gefunden werden. Diese Population zeigte keine Färbung mit einem CMV-Tetramer, was zeigte, dass die Bindung des Keratin 18-Tetramers spezifisch war.

Zusammenfassend lässt sich also sagen, dass für das Keratin 18-Peptid spezifische CD8+ T-Lymphocyten im menschlichen T-Zell Repertoire enthalten sind.

## Patentansprüche

1. In vitro Verfahren zur Identifizierung von MHC-Liganden, mit den Schritten:
a) Bereitstellen einer Probe aus tumorösem und korrespondierendem gesunden Gewebe,
b) Bestimmung des tumorspezifischen Expressionsprofils aus der bereitgestellten Probe,
c) Isolation und Analyse von MHC-Liganden aus der Probe aus tumorösem Gewebe,
d) Abgleich der aus b) und c) gewonnenen Daten, und
e) Identifizierung von MHC-Liganden auf der Basis der abgeglichenen Daten.

2. Verfahren nach Anspruch 1, **dadurch gekennzeichnet, dass** Schritt b) mittels Mikroarray-Analyse und/oder reverser Transkriptase-Polymerase-Kettenreaktion erfolgt.

3. Verfahren nach Anspruch 1 oder 2, **dadurch gekennzeichnet, daß** im Schritt c) die Analyse mittels Massenspektrometer durchgeführt wird.

4. Verfahren nach Anspruch 3, **dadurch gekennzeichnet, dass** im Schritt c) unter Verwendung geeigneter Datenbanken aufgrund des Expressionsprofils potentielle antigene Peptide vorhergesagt werden und das Massenspektrometer auf diese kalibriert wird.

5. Verfahren nach einem der Ansprüche 1 bis 4, **dadurch gekennzeichnet, dass** nach Schritt c) ein weiterer Schritt durchgeführt wird, bei dem die Reaktivität von peripheren Leukocyten, vorzugsweise von T-Lymphocyten gegen die isolierten antigenen Peptide getestet wird.

6. Verfahren nach Anspruch 5, **dadurch gekennzeichnet, dass** die Reaktivitätstestung über die Messung der von Leukocyten synthetisierten Cytokin-mRNA und/oder γ-Interferon-mRNA erfolgt.

7. Verfahren nach einem der Ansprüche 1 bis 4, **dadurch gekennzeichnet, dass** nach Schritt c) ein weiterer Schritt durchgeführt wird, bei dem die Existenz spezifischer T-Lymphocyten nachgewiesen wird.

8. Verfahren nach Anspruch 7, **dadurch gekennzeichnet, dass** der Nachweis spezifischer T-Lymphocyten über die Markierung der Leukocyten mit rekonstituierten Komplexen aus Antigen-präsentierenden Molekülen und antigenem Peptid erfolgt.

## Claims

1. *In vitro* method for identifying of MHC-ligands comprising the following steps:
a) providing a sample of tumorous and corresponding healthy tissue,
b) determining the tumor-specific expression profile in the provided sample,
c) isolation and analysis of MHC-ligands in the sample of tumorous tissue,
d) matching the data obtained in steps b) and c), and
e) identification of MHC-ligands on the basis of the matched data.

2. Method according to claim 1, wherein step b) is performed by microarray analysis and/or reversed transcriptase-polymerase chain reaction.

3. Method according to claim 1 or 2, wherein in step c) analysis is performed by mass spectrometer.

4. Method according to claim 3, wherein in step c) candidate antigenic peptides are predicted on basis of the expression profile using suitable databases, and that the mass spectrometer is calibrated in view of the peptides.

5. Method according to claim 1 to 4, wherein after step c) a further step is performed, in which the reactivity of peripheral leukocytes, preferably T-lymphocytes, is tested against the isolated antigenic peptides.

6. Method according to claim 5, wherein the reactivity test is performed by means of measuring cytokine-mRNA and/or γ-Interferon mRNA synthesised by the leukocytes.

7. Method according to claim 1 to 4, wherein after step c) a further step is performed, in which the presence of specific T-lymphocytes is detected.

8. Method according to claim 7, wherein detection of specific T-lymphocytes is carried out by means of labeling leukocytes with reconstituted complexes of antigen-presenting molecules and antigenic peptide.

## Revendications

1. Procédure *in vitro* d'identification de ligands du CMH, comportant les étapes suivantes :
a) mise à disposition d'un échantillon de tissu tumoral et de tissu correspondant sain;
b) détermination du profil d'expression spécifique à la tumeur sur la base de l'échantillon mis à disposition ;
c) isolation et analyse de ligands du CMH issus de l'échantillon du tissu tumoral ;
d) comparaison des données recueillies dans les étapes a) et c), et
e) identification de ligand du CMH sur la base des données comparées.

2. Procédure selon la revendication 1, **se caractérisant par le fait que** l'étape b) a lieu par analyse par puce ADN et/ou par transcriptase inverse-amplification en chaîne par polymérase.

3. Procédure selon la revendication 1 ou 2 **se caractérisant par le fait qu'**à l'étape c), l'analyse est réalisée à l'aide d'un spectromètre de masse.

4. Procédure selon la revendication 3 **se caractérisant par le fait qu'**à l'étape c), il est, en utilisant des bases de données appropriées et en se basant sur le profil d'expression, possible de prédire les peptides antigéniques potentiels et de calibrer le spectromètre de masse sur ceux-ci.

5. Procédure selon une des revendications 1 à 4 **se caractérisant par le fait qu'**après l'étape c), une autre étape est réalisée, lors de laquelle on teste la réactivité de leucocytes périphériques, de préférence de lymphocytes T contre les peptides antigéniques isolés.

6. Procédure selon la revendication 5 **se caractérisant par le fait que** le test de la réactivité se fait par le biais de la mesure de la mARN de cytokine et/ou de la mARN de gamma-interféron synthétisée par des leucocytes.

7. Procédure selon une des revendications 1 à 4 **se caractérisant par le fait qu'**après l'étape c), une autre étape est réalisée, lors de laquelle on met en évidence l'existence de lymphocytes T spécifiques.

8. Procédure selon une de la revendication 7 **se caractérisant par le fait que** la mise en évidence de lymphocytes T a lieu par le biais du marquage des leucocytes avec des complexes reconstruits issus des molécules présentatrices d'antigène et d'un peptide antigénique.
